# EUROPEAN PATENT APPLICATION

(11) **EP 0 763 738 A1**
(43) Date of publication of application: **19.03.1997**
(21) Application number: 96306029.8
(22) Date of filing: 19.08.1996
(51) Int. Cl.: G01N 33/53, G01N 33/50, G01N 33/569, G01N 33/571, G01N 33/58, G01N 33/543

(54) **Assays for Chlamydia in diluted urine samples**

(30) Priority: 14.09.1995 EP 95306508
(71) Applicant: UNIPATH LIMITED, Basingstoke, Hampshire RG24 OPW (GB)
(72) Inventor: Menton, Jeanette Elizabeth Bridget, Bedford, MK40 2JE (GB); Sheard, Paul Raymond, Wilby, Northampton, NN8 2UB (GB)
(74) Representative: Butler, David John

(57) **Abstract**

Enhanced sensitivity of an assay for a urinary analyte, especially Chlamydia lipopolysaccharide antigen in male urine, is achieved by simple aqueous dilution, e.g. 1:1 with water, of the urine sample, prior to centrifugation of the urine sample and testing of the pellet for the presence of LPS antigen.

## Description

### FIELD OF THE INVENTION

This invention relates to assays, and in particular to methods for preparing urine samples for use in assays.

### BACKGROUND TO THE INVENTION

For many assays of clinical significance, urine is an appropriate sample source. The sample can be obtained easily without discomfort to the patient. However, the overall composition of urine is very variable and this can interfere with the sensitivity and/or accuracy of an assay. The composition of urine can vary very widely, depending on the subject's fluid intake during the previous few hours and on the time of day when the sample is taken.

Urine can contain substantial amounts of components that can bind non-specifically to assay reagents and give rise to misleading or false-positive results.

### GENERAL DESCRIPTION OF THE INVENTION

By the invention we have found that a simple dilution of a urine sample with an aqueous composition, especially distilled or deionised water, can bring about a substantial increase in the sensitivity and/or accuracy of an assay.

The invention provides a method of urine sample preparation, prior to assay thereof for the possible presence of bacterial antigens or the like, preferably by means of an assay involving migration of the sample through a porous carrier wherein an assay result is revealed by specific binding of a labelled reagent in a detection zone on said carrier, wherein the urine sample is diluted with an aqueous fluid. Dilution is preferably conducted before the urine sample is processed to solubilise and recover antigenic material for the purposes of the assay, for example, by filtering or centrifuging the urine sample to obtain a pellet of insoluble material, including bacterial cells, which can be treated with an extraction buffer to free appropriate antigenic material to form the assay analyte.

In general, appropriate dilution is achievable with an amount of aqueous fluid which is from about one half to about twice the volume of the urine sample. About 1:1 dilution of the sample is ideal.

A particular embodiment of the invention is a method of preparing a male urine sample prior to assay thereof for the possible presence of Chlamydia antigens, wherein the sample is diluted with an aqueous fluid to an extent sufficient to cause substantial dissolution of urate salts. Dilution of the sample can be achieved with pure, e.g. de-ionised, water. Alternatively, the diluent can contain one or more ingredients that modify the sample, such as cell lysing agents (e.g. surface active agents) or pH modifiers. Appropriate extraction buffers are described in detail below.

Lipopolysaccharide antigens are important indicators of the presence of certain pathogenic bacteria, notably Chlamydia. It is known that males can act as carriers of Chlamydia, Effective assays for Chlamydia organisms in male subjects are required, especially to facilitate infection contact tracing.

An extraction procedure is used to free lipopolysaccharide antigen from organisms that may be present. A typical extraction procedure will use lysing agents such as detergents. The extraction procedure may involve heating of the sample in the presence of one or more lysing agents, for example to a temperature in the range of 80 to 100°C. After several minutes a sufficient quantity of lipopolysaccharide antigen will have been released from any organisms present to provide basis for an accurate assay.

The reason why sample dilution in accordance with the invention brings about an increase in assay sensitivity is not fully understood. In part, this may be due to dissolution of potentially interfering components. We have observed in some urine samples that the overall salt concentration may be so high that salts such as urate can exist in undissolved form. This is most often observed in male urine samples. These salts tend to be intensely coloured in the crystalline state. For example, urate salts are deep brick-red colour. We have also observed that these salts can bind non-specifically to reagents which are intended to provide a specific assay for an analyte in the urine sample. For example, in an assay for Chlamydia organisms, using an anti-lipopolysaccharide antibody immobilised on a solid phase carrier as a capture agent, we have observed that urate can bind to the capture agent and produce a mis-leading or inaccurate test signal. It is therefore possible that sample dilution, followed by centrifugation for example, to recover a pellet of insoluble material which is separated and further processed to provide the assay sample, may facilitate removal of potentially interfering urate salts. However, this cannot be the whole story. As the experimental results below demonstrate, urine sample dilution provides enhanced assay sensitivity even where the original urine sample does not obviously contain substantial amounts of undissolved salts. In principle, dilution of an assay sample is not normally conducive to enhanced assay sensitivity, but the converse appears to apply in the context of the present invention.

It is already well known that clinical samples often contain irrelevant components which may interfere with the sensitivity of a lipopolysaccharide antigen assay. Cations are a particular nuisance. EP-A-392865 discusses this problem and recommends that divalent cations should be removed from the system, for example, by chelation with agents such as EDTA. EP-A-392865 is particularly concerned with the unwanted presence of divalent cations such as zinc and magnesium. Other cationically-charged materials may also be present in the clinical sample. Any of these cationic materials may interfere with the sensitivity of a lipopolysaccharide antigen assay. We believe that this interference arises because the cations bind to a negatively charged region of the lipopolysaccharide molecule and block an important binding site against which antibodies useful in such an assay are raised.

An important embodiment of the invention is an assay for lipopolysaccharide antigen, conducted in the presence of an anionic polysaccharide in an amount sufficient to reduce interference caused by cations, and wherein the assay sample is a diluted urine sample as set forth above.

Preferably the anionic polysaccharide is a glycosaminoglycan, such as heparin. Alternatives include heparin sulphate and dermatan sulphate.

The invention also provides an aqueous extraction buffer for solubilising antigenic material in bacteria and the like, containing an anionic polysaccharide in an amount sufficient to enhance assay sensitivity, as a diluent for a urine sample.

An important embodiment of the invention is an aqueous extraction buffer for use in solubilising lipopolysaccharide antigen in Chlamydia, containing a glycosoaminoglycan, especially heparin, in an amount sufficient to enhance assay sensitivity, when used as a urine sample diluent. Preferably the buffer contains at least about 0.1 mg/ml heparin. Normally it does not need to contain more than about 10 mg/ml heparin.

When the practice of the invention involves a procedure for extracting solubilised antigenic material from cellular biological material, such as bacteria, wherein the cellular material is treated with an aqueous solution of a surface active agent, this is also conducted in the presence of an anionic polysaccharide. Preferably the surface active agent is zwitterionic. More preferably, the surface active agent is3-(3-chlolamidopropyl)dimethylammonio-1-propanesulfonate (conveniently known as CHAPS) or 3-(3-chlolamidopropyl)dimethylammonio-2-hydroxyl-1-propanesulfonate (conveniently known as CHAPSO), or mixtures thereof.

An especially effective extraction of lipopolysaccharide antigen from Chlamydia species such as Chlamydia trachomitis, C.psittaci and C.twar, is achieved if the extraction is performed using an aqueous solution of heparin and a zwitterionic surface active agent, especially CHAPS and/or CHAPSO.

Preferably, although not essentially, the extraction is conducted at elevated temperature, for example in excess of about 50°C, for a period of time sufficient to solubilise the antigenic material. More preferably, the extraction temperature is at least about 60°C. In general, the extraction temperature need not be greater than about 100°C, and is preferably not greater than about 90°C. Ideally, the extraction temperature is about 80°C. The stage of the extraction conducted at such elevated temperature should generally last for at least about 5 minutes.

Preferably, the quantity of surface active agent in the aqueous extraction medium is at least about 0.1% by weight. Preferably the quantity of surface active agent is not greater than about 2%, and more preferably not greater than about 1% by weight.

The pH of the extraction medium should generally be in the range of about 7.5 to about 9.

The heparin or other anionic polysaccharies can be used either in soluble or insoluble form as desired. Heparin is available commercially in either form. One option is to have the anionic polysaccharide as a soluble component in the extraction buffer and/or urine diluent, or added as a soluble component at a later stage before commencement of the actual assay procedure. Presence as a component in an extraction buffer/diluent is probably the most convenient from the user's point of view. Alternatively, an aqueous sample/extract can be contacted with an insolubilised form of the anionic polysaccharide, for example, heparin bound to a solid surface such as resin beads. The insolubilised anionic polysaccharide can be removed from the aqueous composition before the actual assay is conducted. For example beads can be centrifuged or filtered out. A solid phase of greater bulk, for example a high surface area dipstick on which the heparin is bound can be separated from the aqueous composition by manual extraction or decantation of the liquid. A variety of alternative formats will suggest themselves readily to the skilled reader. The essential objective is that the aqueous composition containing the lipopolysaccharide antigens to be assayed is exposed to the anionic polysaccharide under circumstances which permit the anionic polysaccharide to bind with and in effect, remove from the system at least a proportion of any cationic components present which might interfere with the accuracy of the lipopolysaccharide antigen assay. For any given assay procedure and format the optimum amount and presentation of the anionic polysaccharide can be determined by simple experiment.

In a typical extraction procedure according to the invention, a biological sample in the form of concentrated solid matter from a urine sample obtained from a patient suspected of carrying a Chlamydia infection, for example, is contacted with the extraction medium. Appropriate samples can take the form, for example, of genital, rectal or ocular swabs, or centrifugal pellets from liquids such as early morning urine. Extraction, for example at 80°C for 10 minutes, is followed by a brief period, for example 5 minutes, during which the extraction medium is allowed to cool. Thereafter the extraction medium can be separated from solid matter, for example by removal of the swab and filtration of the solution to provide a sample liquid containing any extracted antigen ready for use in any suitable assay procedure. The assay for the analyte can involve any conventional assay technique, such as radioimmunoassay or enzyme-linked immunoassay. The urine sample is ideal for use in an immunochromatographic assay procedure such as described and claimed in EP-A-291194, especially using a nitrocellulose solid phase and an antibody reagent labelled with a direct particulate label such as coloured latex particles. Dilution and the use of heparin together with CHAPS and/or CHAPSO enhances the sensitivity of a Chlamydia assay which involves anti-lipopolysaccharide antigen antibodies as specific binding reagents.

### EXAMPLES

The following experiments demonstrate the benefits of urine sample dilution in enhancing the sensitivity and/or accuracy of a Chlamydia lipopolysaccharide antigen assay.

### Materials

Dilution with de-ionised water, followed by centrifugation at 3000g for 10 minutes.

Extraction buffer formulation:
0.25 M TRIS pH 8.5 containing
0.85% Sodium chloride
0.25% CHAPSO
5 mM EDTA
1% Bovine serum albumin
1 mg/ml Heparin (SIGMA)

Assay procedure: Heat urine sample in a plastics extraction tube, with filter/dropper cap, at 80°C ± 2°C for 10 minutes. Allow to cool to room temperature for 5 minutes. Place filter cap on extraction tube, and squeeze 5 drops from tube onto a commercially-available "CLEARVIEW Chlamydia" (Unipath Ltd, UK) assay device, as broadly described in EP-A-291194. Assay result revealed as visible line in test window. Line intensity assessed optically in arbitrary units, as a measure of antigen detection relative to a standardised control sample.

### Experiment 1

Samples numbered 1 to 4 (Table 1).

Demonstrates the ability of urine dilution pretreatments to enhance assay performance.

Chlamydia-negative male urine was divided into 2x4 ml aliquots, a) and b).
a) 4ml urine
b) 4ml urine diluted 1:1 with water

Each aliquot was centrifuged at 3000g for 10 minutes, the supernatant removed and the pellet assayed. 600µl extraction buffer was added to resuspend the pellet, and the pellet then transferred to an extraction tube. Samples were tested in the presence of a known amount of Chlamydial antigen (15 µl), as described under "Assay Procedure" above.

Results are detailed in Table 1.

### Experiment 2

Samples numbered 5 and 6 (Table 2).

Demonstrating the ability of urine dilution pretreatment to enhance assay performance from urine samples positive for Chlamydia.

Chlamydia-positive male urines, with brick-red precipitates, were split into 2x4 ml aliquots, a) and b).
a) 4ml urine
b) 4ml urine diluted 1:1 with water

These were processed and assayed as in Experiment 1.

Results are detailed in Table 2.

### Experiment 3

Samples numbered 7 and 8 (Table 2).

Demonstrating the ability of urine dilution pretreatment to inhibit interferences (not necessarily due to urate salts) present in urine samples positive for Chlamydia.

Chlamydia-positive male urines, with no obvious precipitate, were divided into 2x4ml aliquots, a) and b).
a) 4ml urine
b) 4ml urine diluted 1:1 with water

These were processed and assayed as in Experiment 1.

Results are detailed in Table 2.

### Experiment 4

Samples numbered 9 to 14 (Table 3).

Demonstrates the effect of the presence of insoluble salts on the accuracy of an assay if a male urine sample is not diluted prior to testing.

Three Chlamydia-positive samples and three Chlamydia-negative samples were tested without prior dilution of the urine. All samples contained visibly precipitated urate salt. The assays were conducted using commercially-available "CLEARVIEW Chlamydia" assay devices. This product uses a black-coloured latex as a particulate direct lable, which should reveal a positive result as a distinct black line on a white nitrocellulose strip within the device. It is apparent from the data in Table 3 that the precipitated salts in the sample always caused an interfering signal in the test line, to the potential confusion of a user. This effect was eliminated by sample dilution in accordance with the invention.

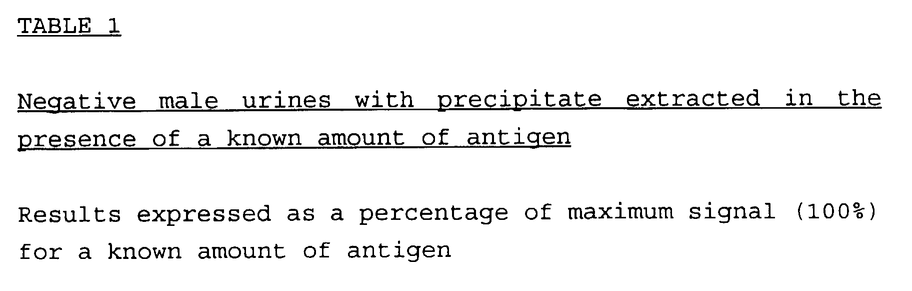

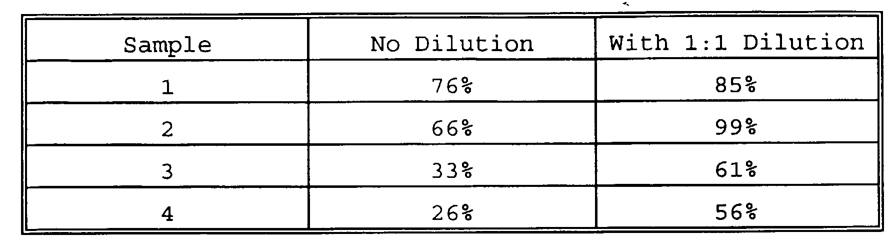

**TABLE 2**

| Chlamydia Positive Male Urine | | | |
|---|---|---|---|
| Results expressed in arbitrary units, representing assay signal line intensity | | | |
| Sample | Presence of precipitate | No Dilution | With 1:1 Dilution |
| 5 | yes - red | 16.2 | 19.4 |
| 6 | | 9.9 | 16.7 |
| 7 | no | 9.7 | 15.6 |
| 8 | | 16.1 | 19.8 |

**TABLE 3**

| Chlamydia Positive/Negative Male Urine | | |
|---|---|---|
| Visual interpretation of test line | | |
| Sample | Chlamydia positive or negative | Visual interpretation of test line |
| 9 | Negative | pink/orange line |
| 10 | Negative | thin orange line |
| 11 | Negative | yellow line |
| 12 | Positive | orange line |
| 13 | Positive | orange/black line |
| 14 | Positive | red/black line |

## Claims

1. A method of urine sample preparation, prior to assay thereof for the possible presence of an analyte, preferably by means of an assay involving migration of the sample through a porous carrier wherein an assay result is revealed by specific binding of a labelled reagent in a detection zone on said carrier, in which preparation method the sample is diluted with an aqueous fluid.

2. A method according to claim 1, wherein the dilution is conducted to an extent sufficient to cause substantial dissolution of salts which may otherwise bind non-specifically in the detection zone during the assay.

3. A method of preparing a male urine sample prior to assay thereof for the possible presence of Chlamydia antigens, wherein the sample is diluted with an aqueous fluid.

4. A method according to any one of the preceding claims, involving dilution of the sample with pure water.

5. A method according to any one of the preceding claims, wherein the dilution is conducted with an amount of aqueous fluid which is from about one half to about twice the volume of the urine sample.

6. A method according to any one of the preceding claims, involving about 1:1 dilution of the sample.

7. A method according to any one of the preceding claims, wherein the dilution is conducted prior to centrifiguration or the like to recover insoluble material which is separated from the liquid and further processed to provide a sample for assay.

8. A method according to any one of the preceding claims, wherein the assay is for a lipopolysaccharide antigen and the urine sample or material separated there from is contacted with an anionic polysaccharide, especially heparin, prior to the assay.

9. A method according to any one of the preceding claims, wherein the aqueous diluent is an extraction buffer to promote release of antigenic material from target organisms that may be present in the urine sample.

10. An in vitro method of detecting the presence of Chlamydia in a male patient, wherein a sample of the patient's urine is diluted with an aqueous composition, preferably water to an extent sufficient to cause substantial dissolution of urate salts, and the diluted sample is tested for the presence therein of Chlamydia lipopolysaccharide antigen by means of an assay wherein a positive test result is revealed by specific binding of a labelled reagent in a zone of a porous carrier, preferably a test strip, the label being a particulate direct label preferably a coloured latex particle.
